(19) Europäisches Patentamt
European Patent Office
Office européen des brevets

(11) Veröffentlichungsnummer: **0 010 256**
**B1**

# (12) EUROPÄISCHE PATENTSCHRIFT

(45) Veröffentlichungstag der Patentschrift: **29.12.82**

(51) Int. Cl.³: **C 07 C 127/15**

(21) Anmeldenummer: **79103887.0**

(22) Anmeldetag: **10.10.79**

## (54) Verfahren zur Herstellung von symmetrischen 1,3-disubstituierten Harnstoffen.

(30) Priorität: **16.10.78 DE 2844962**

(43) Veröffentlichungstag der Anmeldung: **30.04.80 Patentblatt 80/9**

(45) Bekanntmachung des Hinweises auf die Patenterteilung: **29.12.82 Patentblatt 82/52**

(84) Benannte Vertragsstaaten: **AT BE CH DE FR GB IT LU NL SE**

(56) Entgegenhaltungen:
**DD-B-55 003**
**DE-B-2 411 009**
**DE-C-855 551**
**DE-C-896 640**
**US-A-3 937 727**

(73) Patentinhaber: **BASF Aktiengesellschaft, Carl-Bosch-Strasse 38, D-6700 Ludwigshafen (DE)**

(72) Erfinder: **Woerz, Otto, Dr. Dipl.-Chem., Bruesseler Ring 51, D-6700 Ludwigshafen (DE)**
Erfinder: **Block, Ulrich, Dr., Ungsteiner Strasse 14, D-6700 Ludwigshafen (DE)**
Erfinder: **Fischer, Roman, Dr. Dipl.-Chem., Deidesheimer Strasse 1, D-6704 Mutterstadt (DE)**
Erfinder: **Rotermund, Gerhard W., Dr., Landfriedstrasse 3, D-6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 010 256 B1

BUNDESDRUCKEREI BERLIN

Verfahren zur Herstellung von symmetrischen 1,3-disubstituierten Harnstoffen

Die Erfindung betrifft ein Verfahren zur Herstellung von symmetrischen 1,3-disubstituierten Harnstoffen durch Umsetzung von Harnstoff mit primären Aminen.

Die bekannten Verfahren, die auf der Umaminierung von Harnstoff beruhen, unterscheiden sich dadurch, daß sie diskontinuierlich oder kontinuierlich, unter Druck oder drucklos, mit oder ohne Lösungsmittelzusatz, mit oder ohne Aminüberschuß, mit oder ohne Abtrennung und Rückführung von Ausgangsmaterial oder Zwischenprodukten durchgeführt werden.

Von besonderem Interesse ist die diskontinuierliche, drucklose, lösungsmittelfreie, vollständige Umaminierung ohne Aminüberschuß (DE-PS 896 640). Bei der Umaminierung werden jedoch nur dann akzeptable Raum-Zeit-Ausbeuten erzielt, wenn die Reaktionstemperatur zwischen 150 und 200° C liegt. Bei diesen Temperaturen weisen alle Harnstoffe erhebliche Sublimationsdampfdrücke auf, die große technische Probleme verursachen, da pro Mol umgesetzten Harnstoff zwei Mole Ammoniak entstehen. Dieses Abgas wird üblicherweise in Wasser absorbiert, so daß eine 20- bis 25%ige Ammoniaklösung entsteht. Die Entsorgung dieser noch durch Harnstoffe und Amine verunreinigten Lösung ist problematisch.

Schließlich ist noch zu berücksichtigen, daß der Siedepunkt der meisten Amine, die für eine Umaminierung von Interesse sind, weit unter 150 bis 200°C liegt. Das hat zur Folge, daß die Aminkonzentration in der Schmelze zwangsläufig sehr niedrig ist, was zu schlechten Raum-Zeit-Ausbeuten führt. Reaktionszeiten bis zu 30 Stunden werden benötigt (DD-PS 55 003).

Führt man die drucklose und lösungsmittelfreie Umaminierung kontinuierlich durch, wird eine Reaktionskolonne, beispielsweise eine Glockenbodenkolonne, benötigt, die mindestens zwei Temperaturbereiche aufweisen muß (DD-PS 55 003). Auch mit dieser aufwendigen Apparatur wird maximal die Raum-Zeit-Ausbeute der diskontinuierlichen Verfahrensweise erreicht.

Ferner sind diskontinuierliche Verfahren bekannt, bei denen die Umaminierung unter Druck durchgeführt wird. Um disubstituierte Harnstoffe mit einem Umsatz von mindestens 95% zu erhalten, muß das Amin in sehr hohem Überschuß, d. h. in einem Überschuß von mindestens 20 Molen Amin, eingesetzt werden, was für ein großtechnisches Verfahren nicht sinnvoll ist. Um höhere Umsätze zu erzielen, kann ein Überschuß von bis zu 60 Molen Amin erforderlich sein. Diese Verfahren dienen deshalb nur zur Herstellung von monosubstituierten Harnstoffen. Voraussetzung ist, daß der monosubstituierte Harnstoff abgetrennt werden kann und daß nicht umgesetzter Harnstoff und disubstituierter Harnstoff rückgeführt werden können.

Disubstituierte Harnstoffe können durch Druckverfahren diskontinuierlich oder kontinuierlich in hohen Ausbeuten und in guten Raum-Zeit-Ausbeuten nur gewonnen werden, wenn ein Lösungsmittel, das vorzugsweise Wasser ist, zugesetzt wird (JP-AS 42 861/73). Auch bei diesem Verfahren läßt sich der Einsatz von Aminüberschüssen nicht ganz vermeiden; hinzu kommt das Problem der Lösungsmittelabtrennung. Außerdem verursacht der Einsatz von Wasser erhebliche Korrosionsprobleme.

Bei den Verfahren, bei denen an Stelle der flüchtigen Amine ihre Salze, insbesondere die Hydrochloride, eingesetzt werden, muß ebenfalls mit einem Überschuß an Amin gearbeitet werden (DE-AS 2 411 009). Dabei bereiten der hohe Salzanfall und Korrosionsprobleme zusätzliche Schwierigkeiten.

Es wurde gefunden, daß man symmetrische 1,3-disubstituierte Harnstoffe der Formel

$$R-NH-CO-NH-R$$

in der

R einen unverzweigten oder verzweigten Alkylrest mit 1 bis 20 Kohlenstoffatomen, der durch Phenyl oder Phenoxy einfach oder mehrfach substituiert sein kann, einem unverzweigten oder verzweigten Alkoxyalkylrest mit 2 bis 20 Kohlenstoffatomen, einen Monocycloalkylrest mit 3 bis 8 Kohlenstoffatomen oder einen Bicycloalkylrest mit 6 bis 12 Kohlenstoffatomen bedeutet,

durch Umsetzung von Harnstoff mit Aminen der Formel

$$RNH_2$$

in der R die oben genannten Bedeutungen hat, in hoher Raum-Zeit-Ausbeute erhält, wenn man Harnstoff und Amin im Molverhältnis 1 : 2,2 bis 1 : 6 in einem Reaktor mit aufgesetzter Kolonne bei einer Temperatur im Bereich zwischen 150 und 250° C und bei einem Druck im Bereich zwischen 10 und 60 bar umsetzt und das entstehende Ammoniak gegebenenfalls zusammen mit nicht umgesetztem Amin am Kolonnenkopf abnimmt.

Die Besonderheit des erfindungsgemäßen Verfahrens besteht darin, daß das Verfahren nur einen geringen Überschuß an Amin erfordert, in Abwesenheit jeglichen Lösungsmittel abläuft und in einer

einzigen Reaktionsstufe hohe Umsätze bei hoher Raum-Zeit-Ausbeute liefert. Harnstoffe, die auf Grund der hohen Reaktortemperatur sublimieren, werden dabei durch aus der Kolonne in den Reaktor rückfließendes Amin aus den aus dem Reaktor aufsteigenden Dämpfen ausgewaschen, so daß am Kolonnenkopf reines Ammoniak oder ein Gemisch aus Ammoniak und Amin, das leicht destillativ getrennt werden kann, anfallen und keinerlei Entsorgungsprobleme entstehen.

Nach dem erfindungsgemäßen Verfahren lassen sich symmetrische 1,3-disubstituierte Harnstoffe der Formel

$$R-NH-CO-NH-R$$

herstellen, in der R einen unverzweigten oder verzweigten Alkylrest mit 1 bis 20 Kohlenstoffatomen, vorzugsweise 1 bis 12 Kohlenstoffatomen, insbesondere 1 bis 5 Kohlenstoffatomen, einen unverzweigten oder verzweigten Alkoxyalkylrest mit 2 bis 20 Kohlenstoffatomen, vorzugsweise 2 bis 12, insbesondere 2 bis 6 Kohlenstoffatomen, einen Monocycloalkylrest mit 3 bis 8 Kohlenstoffatomen oder einen Bicycloalkylrest mit 6 bis 12 Kohlenstoffatomen bedeutet. Die Alkylreste können gegebenenfalls durch Phenyl oder Phenoxy einfach oder mehrfach substituiert sein.

Als Ausgangsstoffe geeignete Amine der Formel $RNH_2$ sind beispielsweise
Methylamin, Ethylamin, n-Propylamin, Isopropylamin, Cyclopropylamin, n-Butylamin, Isobutylamin, sec-Butylamin, tert-Butylamin, Cyclobutylamin, n-Pentylamin, 1-Methyl-n-butyl-amin, 1-Ethyl-n-propylamin, 3-Methyl-n-butylamin, Neopentylamin, 1,1-Dimethyl-n-propylamin, n-Hexylamin, 1-Methyl-n-butylamin, 1,2-Dimethyl-n-butylamin, 1,4-Dimethyl-n-butylamin, 2,2-Dimethyl-n-butylamin, 1,1-Dimethyl-n-butylamin, n-Heptylamin, 1-Methyl-n-hexylamin, 1-Ethyl-n-pentylamin, 1-n-Propyl-n-butylamin, 1,2-Dimethyl-n-pentylamin, 1,3-Dimethyl-n-pentylamin, 1,4-Dimethyl-n-pentylamin, 2,2-Dimethyl-n-pentylamin, 1,2,2-Trimethyl-n-butylamin, 1,1-Dimethyl-n-hexylamin, n-Octylamin, 1-Methyl-n-heptylamin, 1-Ethyl-n-hexylamin, 1-n-Propyl-n-pentylamin, 1,2-Dimethyl-n-heptylamin, 1,3-Dimethyl-n-heptylamin, 1,4-Dimethyl-n-heptylamin, 2,2-Dimethyl-hexylamin, 1,3-Dimethyl-n-hexylamin, 1,3-Dimethyl-n-hexylamin, 1,4-Dimethyl-n-hexylamin, n-Nonylamin, n-Decylamin, n-Undecylamin, n-Dodecylamin, n-Tridecylamin, n-Tetradecylamin, n-Pentadecylamin, n-Hexadecylamin, n-Heptadecylamin, n-Octadecylamin, n-Nonadecylamin, Eicosylamin, Methoxymethylamin, 2-Methoxy-ethylamin, 2-Methoxy-isopropylamin, 1-Methoxy-n-propylamin, 1-Methoxy-methyl-n-propylamin, 1-Methyl-3-methoxy-n-propylamin, 1-Methoxy-n-butylamin, 1-Methoxy-n-pentylamin, 1-Methoxy-n-hexylamin, 1-Methoxy-n-heptylamin, 1-Methoxy-n-octylamin, 1-Methoxy-n-nonylamin, 1-Methoxy-n-decylamin, 2-Ethoxy-ethylamin, 1-Ethoxy-n-propylamin, 1-Ethoxy-n-butylamin, 1-Ethoxy-n-pentylamin, 1-Ethoxy-n-hexylamin, 1-Ethoxy-n-heptylamin, 1-Ethoxy-n-octylamin, 1-Ethoxy-n-nonylamin, 1-Ethoxy-n-decylamin, 1-n-Propoxy-ethylamin, 1-n-Butoxy-ethylamin, 1-n-Pentoxy-ethylamin, 1-n-Hexoxy-ethylamin, 1-n-Heptoxy-ethylamin, 1-n-Octoxy-ethylamin, 1-n-Nonoxy-ethylamin, 1-n-Decyloxy-ethylamin, 1-n-Undecyloxy-ethylamin, 1-n-Dodecyloxy-ethylamin, 1-n-Tridecyloxy-ethylamin, Benzylamin, -Methylbenzylamin, 2-Phenyl-ethylamin, 3-Phenyl-n-propylamin, 1-Methyl-phenethylamin, 1-Phenyl-n-propylamin, 3-Phenyl-1-methyl-n-propylamin, 2-Isopropoxy-ethylamin, 1-Methyl-2-isopropxy-ethylamin, 3-Isopropoxy-n-propylamin, 1-Isopropoxymethyl-n-propylamin, 2-Phenoxy-ethylamin, 2-Phenoxy-isopropylamin, 3-Phenoxy-n-propylamin, 4-Phenoxy-n-butylamin, Cyclopentylamin, Cyclohexylamin, Cycloheptylamin, Cyclooctylamin, 2-Amino-bicyclo[2.2.1]heptan.
Bevorzugte Amine der Formel $RNH_2$ sind solche, bei denen R unverzweigtes oder verzweigtes Alkyl mit 1 bis 5 Kohlenstoffatomen bedeutet.

Für die Umsetzung geeignete Reaktoren sind Behälter mit außen oder innen liegenden Heizvorrichtungen, die mit Rührvorrichtungen, wie mechanischen Rührern oder Treibstrahlrührern, ausgestattet sein können. Die auf den Reaktor aufgesetzte Kolonne ist mit üblichen Einbauten aus Metall oder Keramik ausgerüstet, wobei vorzugsweise Füllkörper, wie beispielsweise Ringe, Sättel, Formkörper, oder auch Böden, wie beispielsweise Siebböden, Glockenböden, Ventilböden, verwendet werden können. Die Umsetzung kann ebenso in mehreren Reaktoren in Kaskadenanordnung durchgeführt werden.

Harnstoffe und Amin werden im Reaktor in einem Molverhältnis von 1 : 2,2 bis 1 : 6, vorzugsweise von 1 : 3 bis 1 : 4, eingesetzt. Die Umsetzung wird bei einer Temperatur im Bereich zwischen 150 und 250° C, vorzugsweise zwischen 195 und 210° C, und bei einem Druck im Bereich zwischen 10 und 60 bar, vorzugsweise zwischen 15 und 25 bar, durchgeführt.

Um einen Umsatz von mindestens 95% zu erzielen, genügen unter diesen Bedingungen mittlere Verweilzeiten von 0,5 bis 4 Stunden. Der Umsatz kann durch Hochdruck-Flüssigkeitschromatographie analytisch bestimmt werden.

Das bei der Umsetzung entstehende Ammoniak wird am Kolonnenkopf abgezogen. Abhängig vom Siedepunkt und Überschuß des Amins kann am Kolonnenkopf auch ein Gemisch aus Ammoniak und Amin abgenommen werden. Dieses Gemisch kann ohne Schwierigkeiten in einer zweiten Kolonne getrennt und das Amin kann rückgeführt werden.

Das Rücklaufverhältnis der Kolonne kann zwischen 2 : 1 und 20 : 1, vorzugsweise zwischen 4 : 1 und 6 : 1, variiert werden. Die Rektifikation in der aufgesetzten Kolonne wird trotz der hohen Reaktortemperatur nicht durch Sublimation des entstehenden 1,3-disubstituierten Harnstoffs gestört.

Die vorstehenden Ausführungen beziehen sich auf die bevorzugte und in der Technik bedeutsame kontinuierliche Durchführung des erfindungsgemäßen Verfahrens. Es versteht sich jedoch von selbst, daß das Verfahren auch diskontinuierlich ausgeführt werden kann, wobei die genannten Verfahrensbedingungen einzuhalten sind.

Die nach dem erfindungsgemäßen Verfahren herstellbaren Harnstoffe sind wertvolle Ausgangsmaterialien für die Synthese von Farbstoffen und Pflanzenschutzmitteln. So erhält man aus diesen Harnstoffen bei der Einwirkung von Oleum nach dem Verfahren der GB-PS 1 185 439 oder bei der Einwirkung von Schwefeltrioxid mit anschließender Einwirkung von Schwefelsäure nach dem Verfahren der DE-OS 2 424 371 Amidosulfonsäuren.

Die nach diesen Verfahren herstellbaren Verbindungen sind Süßstoffe, insbesondere die N-Cyclohexylamidosulfonsäure bzw. ihre Calcium-, Natrium- oder Kaliumsalze, sowie wertvolle Ausgangsstoffe für die Herstellung von Süßstoffen, Farbstoffen und Schädlingsbekämpfungsmitteln. Beispielsweise können durch Chlorierung, beispielsweise mit Thionylchlorid, die entsprechenden Sulfonsäurechloride hergestellt werden; aus ihnen erhält man durch Umsetzung von Anthranilsäure oder ihren Salzen o-Sulfamidobenzoesäuren. Durch Cyclisierung dieser Stoffe gelangt man zu den 2,1,3-Benzothiadiazin-4-on-2,2-dioxiden, die als Pflanzenschutzmittel Verwendung finden (DE-OS 2 105 687, DE-PS 1 542 836).

## Beispiel 1

Ein beheizter Rührautoklav mit aufgesetzter Füllkörperkolonne (Metall-Raschigringe) wird kontinuierlich mit Harnstoff und Isopropylamin im Molverhältnis 1 : 4 beschickt, d. h. der Isopropylaminüberschuß beträgt 100%. Der Reaktionsdruck beträgt 21 bar, die Reaktionstemperatur 205° C, die mittlere Verweilzeit 4 Stunden und das Rücklaufverhältnis der Kolonne 4,6 : 1. Am Kopf der Kolonne fällt ein Gemisch von Amin und Ammoniak von 0,9 : 1 Mol an. Beim Entspannen des Reaktionsaustrages verdampfen etwa 10% Amin, der Rückstand enthält 99,1% Diisopropylharnstoff und 1,3% Monoisopropylharnstoff (bestimmt durch Hochdruck-Flüssigkeitschromatographie). Das Gemisch schmilzt bei 190° C.

## Beispiel 2

Die im Beispiel 1 beschriebene Apparatur wird kontinuierlich mit Harnstoff und Isopropylamin im Molverhältnis 1 : 4 beschickt.Der Reaktionsdruck beträgt 16 bar, die Reaktionstemperatur 205° C, die mittlere Verweilzeit 9 Stunden und das Rücklaufverhältnis der Kolonne 9 : 1. Am Kopf der Kolonne fällt ein Amin/Ammoniak-Gemisch an. Der Reaktionsaustrag enthält nach dem Entspannen 98,1% Diisopropylharnstoff und 1,2% Monoisopropylharnstoff; das Gemisch schmilzt bei 190° C.

## Beispiel 3

Die im Beispiel 1 beschriebene Apparatur wird kontinuierlich mit Harnstoff und Isopropylamin im Molverhältnis 1 : 4 beschickt. Der Reaktionsdruck beträgt 16 bar, die Reaktionstemperatur 205° C, die mittlere Verweilzeit 5 Stunden und das Rücklaufverhältnis der Kolonne 4,7 : 1. Am Kopf der Kolonne fällt ein Amin/Ammoniak-Gemisch an. Der Reaktionsaustrag enthält nach dem Entspannen 97,2% Diisopropylharnstoff und 2,9% Monoisopropylharnstoff. Das Gemisch schmilzt bei 190° C.

## Beispiel 4

Die in Beispiel 1 beschriebene Apparatur wird kontinuierlich mit Harnstoff und Isopropylamin im Molverhältnis 1 : 2,2 beschickt, d. h. der Isopropyl-Amin-Überschuß beträgt 10%. Der Reaktionsdruck beträgt 16 bar, die Reaktionstemperatur 170 bis 175° C, die mittlere Verweilzeit 4 Stunden. Das Rücklaufverhältnis der Kolonne beträgt ca. 15 : 1. Am Kopf der Kolonne fällt reines Ammoniak an. Der Reaktionsaustrag enthält nach dem Entspannen 90% Diisopropylharnstoff und 10% Monoisopropylharnstoff. Der Schmelzpunkt des Gemisches liegt bei 183 bis 185° C.

Beispiel 5

Eine Umlaufapparatur mit aufgesetzter Füllkörperkolonne (Maschendrahtringe) wird kontinuierlich mit Harnstoff und n-Propylamin im Molverhältnis 1 : 4 beschickt. Der Reaktionsdruck beträgt 21 bar, die Reaktionstemperatur 205°C, die mittlere Verweilzeit 4 Stunden und das Rücklaufverhältnis der Kolonne 5 : 1. Am Kopf der Kolonne fällt ein Amin/Ammoniak-Gemisch an. Der Reaktionsaustrag vom Schmelzpunkt 102 bis 104°C enthält nach dem Entspannen mehr als 95% Di-n-propylharnstoff.

Beispiel 6

Die im Beispiel 1 beschriebene Apparatur wird kontinuierlich mit Harnstoff und Isoamylamin im Molverhältnis 1 : 4 beschickt. Der Reaktionsdruck beträgt 16 bar, die Reaktionstemperatur 225°C, die mittlere Verweilzeit 4 Stunden und das Rücklaufverhältnis der Kolonne 5 : 1. Am Kopf der Kolonne fällt ein Amin/Ammoniak-Gemisch an. Der Reaktionsaustrag enthält 23% Isoamylamin, das abdestilliert wird. Der Destillationsrückstand besteht zu mehr als 95% aus Diisoamylharnstoff; er schmilzt bei 43 bis 46°C.

Beispiel 7

Die im Beispiel 1 beschriebene Apparatur wird kontinuierlich mit Harnstoff und Methylamin im Molverhältnis 1 : 4 beschickt. Der Reaktionsdruck beträgt 20 bar, die Reaktionstemperatur 205°C, die mittlere Verweilzeit 4 Stunden und das Rücklaufverhältnis der Kolonne 5 : 1. Am Kopf der Kolonne fällt ein Amin/Ammoniak-Gemisch an. Der Reaktionsaustrag enthält nach dem Entspannen 98% Dimethylharnstoff und 1% Monomethylharnstoff. Das Produkt schmilzt bei 104 bis 106°C.

**Patentansprüche**

1. Verfahren zur Herstellung von symmetrischen 1,3-disubstituierten Harnstoffen der Formel

$$R-NH-CO-NH-R$$

in der

R einen unverzweigten oder verzweigten Alkylrest mit 1 bis 20 Kohlenstoffatomen, der durch Phenyl oder Phenoxy einfach oder mehrfach substituiert sein kann, einen unverzweigten oder verzweigten Alkoxyalkylrest mit 2 bis 20 Kohlenstoffatomen, einen Monocycloalkylrest mit 3 bis 8 Kohlenstoffatomen oder einen Bicycloalkylrest mit 6 bis 12 Kohlenstoffatomen bedeutet,

durch Umsetzung von Harnstoff mit Aminen der Formel

$$RNH_2$$

in der R die oben genannten Bedeutungen hat, dadurch gekennzeichnet, daß man Harnstoff und Amin im Molverhältnis 1 : 2,2 bis 1 : 6 in einem Reaktor mit aufgesetzter Kolonne bei einer Temperatur im Bereich zwischen 150 und 250°C und bei einem Druck im Bereich zwischen 10 und 60 bar umsetzt und das entstehende Ammoniak gegebenenfalls zusammen mit nicht umgesetztem Amin am Kolonnenkopf abnimmt.

2. Verfahren nach Anspruch 1, dadurch gekennzeichnet, daß man die Umsetzung von Harnstoff mit Amin kontinuierlich durchführt.

**Claims**

1. A process for the preparation of a symmetrical 1,3-disubstituted urea of the formula

$$R-NH-CO-NH-R$$

where R is unbranched or branched alkyl of 1 to 20 carbon atoms, which may be monosubstituted or polysubstituted by phenyl or phenoxy, or is unbranched or branched alkoxyalkyl of 2 to 20 carbon atoms or is monocycloalkyl of 3 to 8 carbon atoms or is bicycloalkyl of 6 to 12 carbon atoms, by reacting

urea with an amine of the formula

$RNH_2$

where R has the above meanings, wherein urea and an amine are reacted in the molar ratio of from 1 : 2.2 to 1 : 6 in a reactor fitted with a column, at between 150 and 250°C under a pressure of between 10 and 60 bar, and the resulting ammonia, together with any unconverted amine, is taken off at the top of the column.

2. A process as claimed in claim 1, wherein the reaction of urea with the amine is carried out continuously.

**Revendications**

1. Procédé de préparation d'urées 1,3-disubstituées symétriques de la formule

$R-HN-CO-NH-R$

dans laquelle

R désigne un radical alcoyle en $C_1$ à $C_{20}$ à chaîne droite ou ramifiée, pouvant être substitué par un ou plusieurs groupes phényle ou phénoxy, ou un radical alcoxyalcoyle en $C_2$ à $C_{20}$ à chaîne droite ou ramifiée ou un groupe mono-cyclo-alcoyle en $C_3$ à $C_8$ ou un groupe bicyclo-alcoyle en $C_6$ à $C_{12}$,

par réaction de l'urée avec une amine de la formule

$R-NH_2$

dans laquelle R possède l'une des significations définies ci-dessus, caractérisé en ce que l'on fait réagir l'urée et l'amine dans une proportion molaire comprise entre 1 : 2,2 et 1 : 6 dans un réacteur surmonté d'une colonne à une température comprise dans la gamme de 150 à 250°C et sous une pression comprise dans la gamme de 10 à 60 bars, l'ammoniac formé étant soutiré en tête de la colonne, éventuellement avec l'amine non transformée.

2. Procédé suivant la revendication 1, caractérisé en ce que la réaction de l'urée et de l'amine est réalisée en continu.